# EUROPÄISCHE PATENTSCHRIFT

(11) **EP 0 632 810 B1**
(45) Veröffentlichungstag und Bekanntmachung des Hinweises auf die Patenterteilung: **29.11.2000**
(21) Anmeldenummer: 94905703.8
(22) Anmeldetag: 25.01.1994
(51) Int. Cl.: C07D 495/04, C07K 1/00, G01N 33/53

(54) **NEUES BIOTINYLIERUNGSREAGENZ**
NEW BIOTINYLATION REAGENT
NOUVEAU REACTIF DE BIOTINYLATION

(30) Priorität: 27.01.1993 DE 4302241
(43) Veröffentlichungstag der Anmeldung: 11.01.1995
(73) Patentinhaber: Roche Diagnostics GmbH, 68305 Mannheim (DE)
(72) Erfinder: HUBER, Erasmus, D-86923 Finning (DE); ZINK, Bruno, D-82449 Uffing (DE); LENZ, Helmut, D-82327 Tutzing (DE); HOESS, Eva, D-82319 Starnberg (DE)
(74) Vertreter: Huber, Bernhard, Dipl.-Chem.
(86) Internationale Anmeldenummer: EP9400195
(87) Internationale Veröffentlichungsnummer: WO9417072

(56) Entgegenhaltungen:
- EP-A- 0 155 854
- EP-A- 0 156 287
- EP-A- 0 574 000
- DE-A- 3 629 194
- BIOCHEMISTRY Bd. 23, Nr. 12 , 1984 , EASTON, PA US Seiten 2547 - 2553 K. HOFMANN ET AL 'Synthesis of biotinylated and dethiobiotinylated insulins'
- BIOCHEMISTRY Bd. 26, Nr. 23 , 1987 , EASTON, PA US Seiten 7384 - 7390 K. HOFMANN ET AL 'The rat liver insulin receptor'
- JOURNAL OF THE AMERICAN CHEMICAL SOCIETY. Bd. 112, Nr. 8 , 1990 , GASTON, PA US Seiten 3249 - 3250 D. HOYER ET AL 'A new strategy for selective protein cleavage'

## Beschreibung

Die vorliegende Erfindung betrifft ein neues Biotinylierungsreagenz, ein Verfahren zu seiner Herstellung, seine Verwendung zur Biotinylierung von Substanzen mit freien primären oder/und sekundären Aminogruppen und ein Konjugat, welches durch Reaktion des Biotinylierungsreagenz mit Aminogruppen-haltigen Substanzen entsteht. Weiterhin betrifft die Erfindung ein Verfahren zur Bestimmung eines Analyten unter Verwendung des erfindungsgemäßen Konjugats sowie ein Reagenz zur Bestimmung eines Analyten, das ein erfindungsgemäßes Konjugat enthält.

Das aus den Bindepartnern Biotin und Streptavidin/Avidin bestehende Bindungspaar ist für Verfahren auf dem Gebiet der Medizin, Biochemie, Molekularbiologie und Immunologie von sehr großer Bedeutung. Der Grund hierfür ist, daß Biotin oder ein Biotinderivat, das an eine andere Substanz gebunden ist, immer noch zu einer hochaffinen Wechselwirkung mit Streptavidin bzw. Avidin in der Lage ist und der auf diese Weise entstehende Biotin-Streptavidin/Avidin-Komplex ein hervorragendes Nachweissystem für biologische Substanzen darstellt.

Die Biotinylierung unterschiedlicher Substanzen, wie etwa Proteine, Peptide, Nukleinsäuren, Nukleotide oder Haptene mit einem Biotinylierungsreagenz ist seit langem bekannt. Heute sind Biotin-Aktivester, insbesondere Biotin-N-hydroxysuccinimidester (Biotin-NHS) (E.A. Bayer und M. Wilchek in Methods of Biochemical Analysis, Vol. 26 (Editor: D. Glick), Interscience Publication, J. Wiley & Sons, S. 2-45) und Biotintin-ε-amino-capronsäure-N-hydroxysuccinimidester (Biotin-X-NHS) (R.H. Burdon und P.H. von Knippenberg in Laboratory Techniques in Biochemistry and Molecular Biology, Vol. 15 (Editor: P. Tijssen), Elsevier, S. 23-38; S.M. Costello et al., Clin.Chem. 25/9 (1979), S. 1572-1580) die gebräuchlichsten Biotinylierungsreagenzien. Biotin kann in Form dieser Aktivester über stabile Amidbindungen an einen Reaktionspartner mit primären oder/und sekundären Aminofunktionen gekuppelt werden, ohne daß die für seine Bindefähigkeit an Avidin/Streptavidin relevante Heterobicycluseinheit strukturell beeinträchtigt wird. Da insbesondere primäre Aminofunktionen in Form von ε-Aminogruppen der Lysinseitenketten in praktisch jedem Protein vorkommen, hat sich die Aminolyse der Aktivester als Methode der Wahl zur Biotinylierung von Proteinen, wie etwa Antikörpern oder enzymatisch aktiven Proteinen, aber auch von Haptenen, wie etwa Steroidhormonen (L.-X. Tiefenauer und R.Y. Andres, J. Steroid Biochem. 35 (1990), S. 633-639) etabliert. Daher werden Biotin-NHS und Biotin-X-NHS auch von einer Vielzahl von Herstellern von Feinchemikalien und Bioreagenzien vertrieben.

Ebenfalls seit langem bekannt ist die Tatsache, daß es sich bei Biotinylierungsreagenzien als vorteilhaft erweist, zwischen der Biotineinheit und dem zu biotinylierenden Reaktionspartner einen Abstandshalter bzw. "Spacer" einzuführen (N.M. Green et al., Biochem. J. 125 (1971), S. 781-791). Dabei haben sich insbesondere Spacer mit amphiphilen Strukturen besonders bewährt (siehe z.B. EP 0 410 280 A1; EP 0 451 810 A1).

Ein Nachteil der bisher bekannten Biotin-Aktivester besteht jedoch darin, daß sie in fast allen gebräuchlichen organischen Lösungsmitteln nur schwer- oder teilweise sogar unlöslich sind. Beispielsweise sind Biotin-NHS und insbesondere Biotin-X-NHS in organischen Lösungsmitteln mit Ausnahme von DMSO und DMF nur mäßig löslich. Auch in Wasser und wäßrigen Pufferlösungen zeigen die beiden oben genannten Biotinylierungsreagenzien ebenfalls eine nur mäßige Löslichkeit. Das kommerziell erhältliche Biotinylierungsreagenz Sulfo-NHS-LC-Biotin (I.M. Grumbach und R.W. Veh, J. Immunol. Meth. 140 (1991), S. 205-210), bei dem es sich um eine sulfonierte Variante von Biotin-X-NHS handelt, zeigt zwar eine gute Löslichkeit in einem wäßrigen Medium, ist aber wiederum in organischen Lösungsmitteln in den benötigten Konzentrationen nicht löslich. Letzteres wirkt sich insbesondere bei der Synthese von Hapten-Biotin-Konjugaten unvorteilhaft aus, die vorzugsweise in organischen Lösungsmitteln, wie etwa Dioxan, DMF etc. durchgeführt wird (L.-X. Tiefenauer und R.Y. Andres, J. Steroid Biochem. 35 (1990), S. 633-639; EP 0 451 810 A1).

Die europäische Patentanmeldung EP-A-0 156 287 beschreibt ein Biotinylierungsreagenz der Formel:

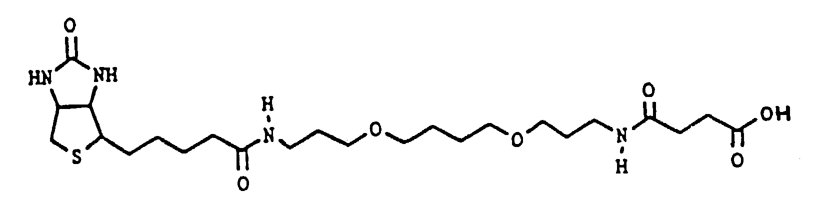

Diese Substanz kann mit Aminomethyltrioxalen-Hydrochlorid bei Raumtemperatur in Gegenwart eines wasserlöslichen Carbodiimids zur Herstellung eines biotinylierten Psoralenderivats umgesetzt werden. Auf Seite 20, Zeilen 23-25 von EP-A-0 156 287 wird erwähnt, daß alternativ die Carboxylgruppe der obigen Substanz in einen Aktivester wie etwa N-Hydroxysuccinimid überführt und anschließend mit Aminomethyltrioxalen-Hydrochlorid umgesetzt werden kann. Eine derartige Umsetzung wird aber nicht in Form eines Beispiels beschrieben, da sich die aus der obigen Verbindung durch Reaktion mit N-Hydroxysuccinimid entstehende Substanz mit der Formel: 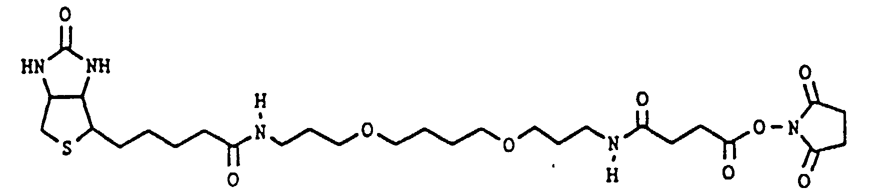 aufgrund der allgemeine Instabilität von Bernsteinsäureamid-Aktivestern in einer intramolekularen Reaktion spontan unter Abspaltung von Hydroxysuccinimid und Cyclisierung in die folgende Substanz: 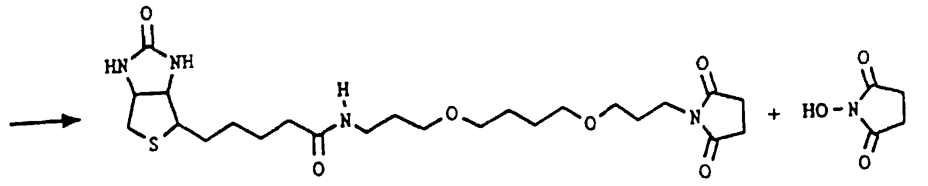 zersetzen dürfte. Somit wird gemäß der Lehre von EP-A 0 156 287 kein stabiler und isolierbarer Biotin-Aktivester erhalten.

Die der vorliegenden Erfindung zugrundeliegende Aufgabe bestand somit darin, ein Biotinylierungsreagenz bereitzustellen, bei dem die oben genannten Nachteile des Standes der Technik zumindest teilweise beseitigt werden. Insbesondere sollte ein Biotinylierungsreagenz mit einer besseren Löslichkeit als bisher bekannte Reagenzien bereitgestellt werden.

Die erfindungsgemäße Aufgabe wird gelöst durch eine Verbindung der allgemeinen Formel I: 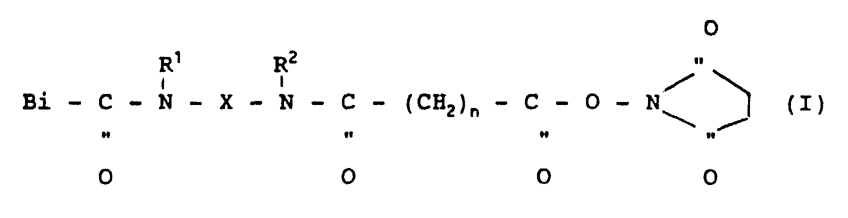 worin
Bi einen durch Abspaltung einer Carboxylgruppe von Biotin oder einem Biotinderivat stammenden Rest bedeutet,
R¹ und R² unabhängig voneinander Wasserstoff oder C₁-C₄-Alkyl bedeuten,
n eine ganze Zahl von 4 bis 10 bedeutet, und
X einen durch ein oder mehrere O- oder/und S-Atome substituierten Alkylenrest mit einer Kettenlänge von 5 bis 20 Atomen bedeutet.

In der Formel I bedeutet Bi eine durch Abspaltung einer Carboxylgruppe von Biotin oder einem Biotinderivat stammenden Rest. Vorzugsweise bedeutet Bi einen von Biotin, Dethiobiotin oder Iminobiotin, besonders bevorzugt von Biotin stammenden Rest.
R¹ und R² bedeuten unabhängig voneinander Wasserstoff oder C₁-C₄-Alkyl, vorzugsweise bedeuten R¹ und R² Wasserstoff oder Methyl, besonders bevorzugt Wasserstoff.
n bedeutet eine ganze Zahl von 4 bis 10, besonders bevorzugt eine ganze Zahl von 6 bis 8.
X bedeutet einen durch ein oder mehrere O- oder/und S-Atome, vorzugsweise durch ein oder mehrere O-Atome substituierten Alkylenrest mit einer Kettenlänge von 5 bis 20 Atomen, vorzugsweise von 5 bis 11 Atomen. Günstigerweise ist X ein unverzweigter Rest, er kann jedoch auch Seitengruppen wie etwa Methyl enthalten. Die Anzahl der O- oder/und S-Atome im Rest X ist vorzugsweise 1 - 5, besonders bevorzugt 1 - 3 und am meisten bevorzugt 2. Weiterhin bedeutet X vorzugsweise ein Polyalkylenoxidderivat, das z.B. Butylenoxid- (C₄H₈O-), Propylenoxid- (C₃H₆O-) und besonders bevorzugt Ethylenoxid-(C₂H₄O-) Einheiten enthält. Besonders bevorzugt stellt X einen Rest (CH₂-CH₂O)ₙCH₂-CH₂ dar, wobei n eine ganze Zahl von 1 bis 5, besonders bevorzugt eine ganze Zahl von 1 bis 3, am meisten bevorzugt 2 bedeutet.

Ein ganz besonders bevorzugter Gegenstand der vorliegenden Erfindung ist Biotinoyl-amino-3,6-dioxaoctanyl-aminocarbonylheptansäure-N-hydroxysuccinimidester (Biotin-DADOO-DSS bzw. Biotin-DDS) mit der Formel:

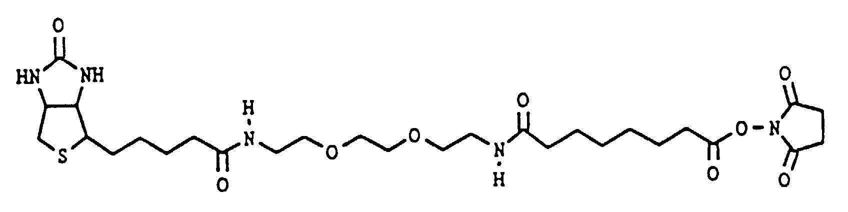

Die erfindungsgemäßen Verbindungen sind hervorragend als Biotinylierungsreagenzien geeignet, da sie eine Kupplung von Biotin oder Biotinderivaten in einem Ein-Schritt-Verfahren ohne Zugabe weiterer Reagenzien an primäre oder/und sekundäre Aminogruppen von Reaktionspartnern wie Proteinen, Peptiden, Nukleinsäuren, Nukleotiden, Haptenen etc. ermöglichen. Die Einführung von Biotingruppen gelingt dabei mit einer überraschend hohen Ausbeute, was bisher mit keinem anderen bekannten Reagenz möglich war.

Weiterhin zeigen die erfindungsgemäßen Verbindungen überraschenderweise ein wesentlich breiteres Löslichkeitsspektrum in Lösungsmitteln wie etwa Wasser und organischen Lösungsmitteln als die Biotinylierungsreagenzien des Standes der Technik, so daß die erfindungsgemäßen Substanzen universeller einsetzbar sind.

Ein weiterer Gegenstand der vorliegenden Erfindung ist ein Verfahren zur Herstellung der neuen Biotinylierungsreagenzien, welches dadurch gekennzeichnet ist, daß man eine Verbindung der allgemeinen Formel (II): 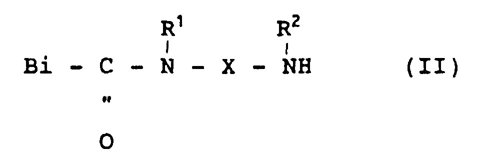 in einem wasserfreien aprotischen Lösungsmittel und in Gegenwart einer Base mit einer Verbindung der allgemeinen Formel (III): 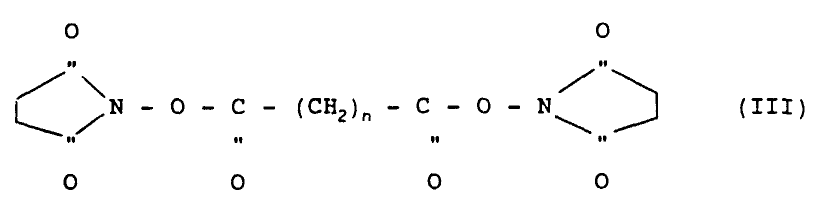 umsetzt und das Reaktionsprodukt gewinnt, wobei Bi, R¹, R², X und n die in Formel (I) angegebene Bedeutung besitzen.

Ein Beispiel für eine Verbindung der Formel (II) ist das kommerziell erhältliche Biotinoyl-1,8-diamino-3,6-dioxaoctan (Biotin-DADOO, Boehringer Mannheim GmbH, Katalog Nr. 1112047). Ein Beispiel für eine Verbindung der Formel (III) ist das kommerziell erhältliche Disuccinimidylsuberat (DSS, Boehringer Mannheim GmbH, Katalog Nr. 1081730). Durch Reaktion dieser beiden Substanzen entsteht die erfindungsgemäß besonders bevorzugte Verbindung Biotin-DADOO-DSS.

Die Reaktion wird in einem wasserfreien aprotischen Lösungsmittel, vorzugsweise DMF, in Gegenwart einer Base, vorzugsweise einem tertiären Amin (z.B. Triethylamin) und vorzugsweise bei Raumtemperatur durchgeführt. Das Produkt kann durch Abziehen des Lösungsmittels im Vakuum, Digerieren in Wasser und Filtration von überschüssigem Ausgangsprodukt aus dem Reaktionsgemisch isoliert werden. Eine weitere Aufreinigung kann gegebenenfalls durch eine Chromatographie über Kieselgel erfolgen.

Noch ein weiterer Gegenstand der vorliegenden Erfindung ist die Verwendung der neuen Biotinylierungsreagenzien zur Biotinylierung von Substanzen mit freien primären oder/und sekundären Aminogruppen. Bevorzugte Beispiele für derartige Substanzen sind Proteine, wie etwa Antikörper (z.B. vollständige (monoklonale oder polyklonale) Antikörper oder Antikörperfragmente), enzymatisch aktive Proteine (z.B. Peroxidase, β-Galactosidase oder alkalische Phosphatase), Nukleinsäuren (z.B. DNA, RNA, Oligonukleotide), Nukleotide (z.B. UTP, dUTP, ddUTP, ATP, dATP, ddATP) oder Haptene (z.B. Fluorescein, Steroidhormone wie etwa Estradiol, Digoxigenin etc.) oder Peptide.

Noch ein weiterer Gegenstand der vorliegenden Erfindung ist ein Konjugat der allgemeinen Formel (IV): wobei 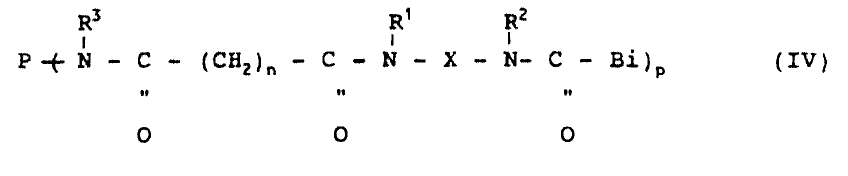
P eine Substanz bedeutet, die mindestens eine primäre oder/und sekundäre Aminogruppe NHR³ besitzt,
R³ Wasserstoff oder/und einen gegebenenfalls substituierten Alkylrest bedeutet,
p eine ganze Zahl von ≥ 1 bedeutet und
Bi, R¹, R², X und n wie in Formel I definiert sind.

Die Substanz P ist vorzugsweise ein Protein, ein Peptid, eine Nukleinsäure, ein Nukleotid oder ein Hapten, besonders bevorzugt ein Protein, wie etwa ein Antikörper oder ein enzymatisch aktives Protein. Das dem Biotinylierungsgrad der Substanz P entsprechende Symbol p bedeutet eine ganze Zahl ≥ 1 und hängt von der Struktur der Substanz P, d.h. von der Anzahl reaktiver Aminogruppen der Substanz P, ab. So kann p bei Peptiden oder Nukleotiden, die nur eine freie Aminogruppe besitzen, 1 bedeuten, während im Falle von Makromolekülen wie Proteinen oder Nukleinsäuren p eine Zahl von bis zu 100, vorzugsweise bis zu 20, besonders bevorzugt bis zu 10 bedeuten kann. Für einen Antikörper ist z.B. ein Biotinylierungsgrad von 1:1 bis 1:10, insbesondere von 1:2 bis 1:7,5 bevorzugt.

Die Erfindung betrifft auch ein Verfahren zur Bestimmung eines Analyten unter Verwendung eines erfindungsgemäßen Konjugats. Es ist ersichtlich, daß derartige Verfahren auf an sich bekannte Weise durchgeführt werden können und sich von den Verfahren des Standes der Technik nur durch die Verwendung der neuen Konjugate unterscheiden.

Ein überraschender Vorteil der Verwendung der erfindungsgemässen Biotinkonjugate bei einem Verfahren zur Bestimmung eines Analyten im Vergleich zu Konjugaten des Standes der Technik besteht darin, daß die mit dem neuen Biotinylierungsreagenz (Biotin-DADOO-DSS) hergestellten Konjugate in einem Elektrochemilumineszenzsystem und auch bei allen anderen Immuntests, bei denen kugelförmige Festphasen verwendet werden, eine Performance ergeben, die um den Faktor 1,3 besser als die mit Biotin-X-NHS erhaltene und etwa um den Faktor 3,5 besser als die mit Biotin-NHS erhaltene Performance ist.

Ein Gegenstand der Erfindung ist schließlich auch ein Reagenz zur Bestimmung eines Analyten, das ein erfindungsgemäßes Konjugat enthält. Das erfindungsgemäße Reagenz ist vorzugsweise in Form einer Lösung, eines Pulvers oder eines Lyophilisats.

Die Erfindung soll weiterhin durch die folgenden Beispiele veranschaulicht werden.

### Beispiel 1

### Synthese von Biotinoyl-amino-3,6-dioxaoctanylaminocarbonylheptansäure-N-hydroxysuccinimidester (Biotin-DADOO-DSS bzw. Biotin-DDS)

Eine Lösung von 561 mg (1,5 mmol) Biotinoyl-1,8-diamino-3,6-dioxaoctan (Biotin-DADOO, Boehringer Mannheim, Katalog Nr. 1112074) in 25 ml frisch destilliertem Dimethylformamid (DMF) wird mit 0,21 ml (1,5 mmol) Triethylamin versetzt und unter Rühren langsam in eine Lösung von 5,52 g (15 mmol) Disuccinimidylsuberat (DSS, Boehringer Mannheim, Katalog Nr. 1081730) in 50 ml frisch destilliertem DMF getropft. Man läßt 18 h bei Raumtemperatur rühren. Danach wird die Lösung im Ölpumpenvakuum am Rotationsverdampfer eingedampft, der halbfeste Rückstand mit ca. 70 ml Wasser digeriert und das überschüssige DSS durch Filtration abgetrennt. Die klare Lösung wird lyophilisiert und das Lyophilisat anschließend mit wenig THF digeriert. Nach Absaugen des festen Produkts wird am Hochvakuum getrocknet.
- Ausbeute:: 620 mg farbloses Pulver.
- DC:: Kieselgel 60 (Merck); Essigester/Eisessig/Wasser 6/3/1 (v/v/v); Besprühen mit einer Mischung 1/1 (v/v) von A) 2 % H₂SO₄ in Ethanol und B) 0,2 % 4-Dimethylamino-zimtaldehyd in Ethanol und anschließendes 10 minütiges Trocknen bei 100°C ergibt himbeerrote Flecken des Produkts bei R_{f} = 0,51.
- ¹H-NMR(d₆-DMSO):: δ = 1,10-1,80 (m, 14 H), 2,05 (t, 4H), 2,65 (t, 2H), 2,80 (s, 4H), 2,60-3,60 (m, 11H), 3,50 (s, 4H), 4,05-4,40 (m, 2H), 6,36 (d,br, 2H), 7,80 (t,br, 2H).

### Beispiel 2

### Biotinylierung von Kaninchen-IgG

100 mg Kaninchen IgG werden in einer Konzentration von 5 mg/ml in PBS-Puffer pH 8,5 gelöst und mit der 10fach molaren Menge an Biotin-DSS in DMF (c = 50 mg/ml) versetzt. Die Lösung wird 1 h bei Raumtemperatur gerührt. Danach stoppt man die Reaktion durch Zugabe von 10 ml 0,1 mol/l Ammoniumacetat-Lösung. Man dialysiert 16 h gegen PBS-Puffer pH 7,5 und lyophilisiert abschließend.

### Beispiel 3

### Löslichkeiten von Biotinylierungsreagenzien

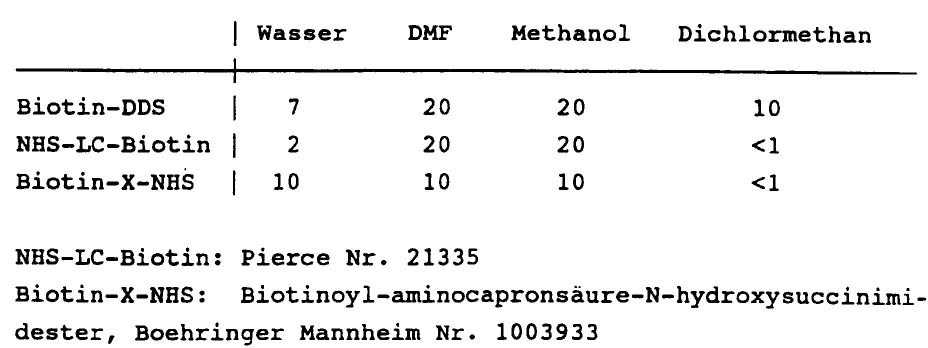

Die Ergebnisse der Löslichkeitsversuche von Biotin-DADOO-DSS im Vergleich mit Biotinylierungsreagenzien des Standes der Technik zeigen, daß das erfindungsgemäße Biotinylierungsreagenz ein erheblich breiteres Löslichkeitsspektrum als bekannte Verbindungen besitzt. Vor allem zeigt es eine gute Löslichkeit sowohl in stark polaren (Wasser) als auch in unpolaren (Dichlormethan) Lösungsmitteln.

### Beispiel 4

### Biotinylierung von monoklonalen Antikörpern gegen Thyreotropin (TSH)

Der monoklonale Antikörper Anti-TSH M 1.20 (MAK 〈hTSH〉 M-I20PK30, ECACC 87122202) wird in 0,1 mol/l Kaliumphosphatpuffer pH 8,4 in einer Konzentration von 10 mg/ml gelöst. Nach Zugabe der 3,5-fachen bzw. 8-fachen molaren Menge an Biotin-DDS (bzw. zum Vergleich Biotin-X-NHS oder Biotin-NHS, jeweils gelöst in Dimethylsulfoxid in einer Konzentration von 9,5 mg/ml) wird 90 Min. bei 25°C gerührt und die Reaktion anschließend durch Zugabe von 10 µl 1 mol/l Lysin gestoppt. Nach Dialyse gegen 25 mmol/l Kaliumphosphat/50 mmol/l Natriumchlorid pH 7,0 wird das Produkt lyophilisiert.

### Beispiel 5

### Immunologische Bestimmung von TSH und Hepatitis B-Oberflächenantigen (HBs) über Elektrochemilumineszenz

### a) Herstellung Ruthenium-markierter F(ab')₂-Fragmente

Der Nachweis von TSH und HBs erfolgt bei der Elektrochemilumineszenz-Bestimmung über Ruthenium-markierte Antikörperfragmente gegen TSH bzw. HBs. Zur Herstellung dieser Fragmente werden 100 mg des monoklonalen Antikörpers Anti-〈TSH〉-M-A8 (Boehringer Mannheim GmbH, Kat.-Nr. 1367978) bzw. Anti-〈HBs〉 M-5A10 (Boehringer Mannheim GmbH, Katalog Nr. 112 4986-122) nach Johnstone und Thorpe (Immunochemistry in Practice, S. 61, Blackwell Scientific, 1987) mit Pepsin gespalten und die resultierenden F(ab')₂-Fragmente durch Chromatographie an Sepharose S 200 HR (Pharmacia LKB) gereinigt. Jeweils 5 mg dieser gereinigten Fragmente werden in 1 ml 0,15 mol/l Kaliumphosphatpuffer/0,15 mol/l Natriumchlorid pH 7,8 gelöst. Unmittelbar vor Gebrauch werden 5 mg Ru(bpy)₃²⁺-NHS (Ruthenium (2,2'-Bipyridyl)₂(4-[3-(N-hydroxysuccinimidylcarboxy)-propyl]-4'-methyl-2,2'-bipyridin)²⁺) (Herstellung gemäß EP-A 0 265 519) in 0,75 ml wasserfreiem Dimethylsulfoxid gelöst. Dieser aktive Ester wird in einem Molverhältnis von 7,5 : 1 von Ester zu F(ab')₂-Fragment (entsprechend 0,396 mg Ru(bpy)₃²+²+-NHS/5 mg F(ab')₂) unter Rühren zupipettiert. Anschließend wird 60 Min. bei 25°C inkubiert und die Umsetzung durch Zugabe von 10 µl 1 mol/l Lysin gestoppt. Nach Dialyse gegen 25 mmol/l Kaliumphosphatpuffer/ 0,1 mol/l Natriumchlorid pH 7,0 für 24 h wird das Produkt lyophilisiert.

### b) Durchführung der Elektrochemilumineszenz-Messung

Die gemäß Beispiel 4 erhaltenen biotinylierten Antikörper Anti〈TSH〉M 1.20 sowie in analoger Weise hergestellte Antikörper gegen das Hepatitis B-Oberflächenantigen Anti〈HBs〉M-5A10 werden in einer Konzentration von 2,25 µg/ml in Inkubationspuffer, welcher 20 % Kaninchenserum enthält, gelöst.

### Inkubationspuffer:

- 112 mmol/l: KH₂PO₄
- 88 mmol/l: K2HPO4
- 0,05 mmol/l: Natriumchlorid
- 6,5 mmol/l: NaN₃
- 0,8 µmol/l: Triton X-100
- 0,4 mmol/l: Tween 20
- 100 mmol/l: Tripropylamin
gelöst in Wasser (pH 7,55 - 7,65).

Die Ruthenium-markierten F(ab')₂-Fragmente des monoklonalen Antikörpers Anti〈TSH〉M-A8 bzw. Anti〈HBs〉M-5A10 werden in einer Konzentration von 1 µg/ml in Inkubationspuffer mit 20 % Kaninchenserumzusatz gelöst. Streptavidin-Magnetpartikel (superparamagnetische Polystyrolpartikel mit 2,8 µm Durchmesser, Dynal A.S., Oslo, Norwegen) werden in einer Konzentration von 600 µg/ml Inkubationspuffer/20 % Kaninchenserum suspendiert und durch Schütteln in homogener Verteilung gehalten. Für die Elektrochemilumineszenz-Messung werden in je 1 Polystyrolröhrchen pipettiert:
- 50 µl: Magnetpartikel-Suspension
- 50 µl: biotinylierter Antikörper
- 50 µl: Ruthenium-markierte F(ab')₂-Fragmente
- 50 µl: TSH-Standardlösung (60 µU/ml) bzw. HBsAg-Standardlösung 12 Einheiten/ml, Boehringer Mannheim GmbH, Katalog Nr. 112 4986-122)

Die Röhrchen werden 16 Min. bei Raumtemperatur unter Schütteln inkubiert. Anschließend werden 500 µl Inkubationspuffer hinzugefügt und nach Durchmischung in einem Aliquot von 225 µl die an die Magnetpartikel gebundene Rutheniummarkierung mit dem Elektrochemilumineszenz-Meßgerät Origen 1.0 der Firma Igen Inc., Rockville, USA (siehe auch G.F. Blackburn et al., Clinical Chemistry 37 (1991), 1534) bestimmt. Das Ergebnis dieser Messungen zeigt die folgende Tabelle.

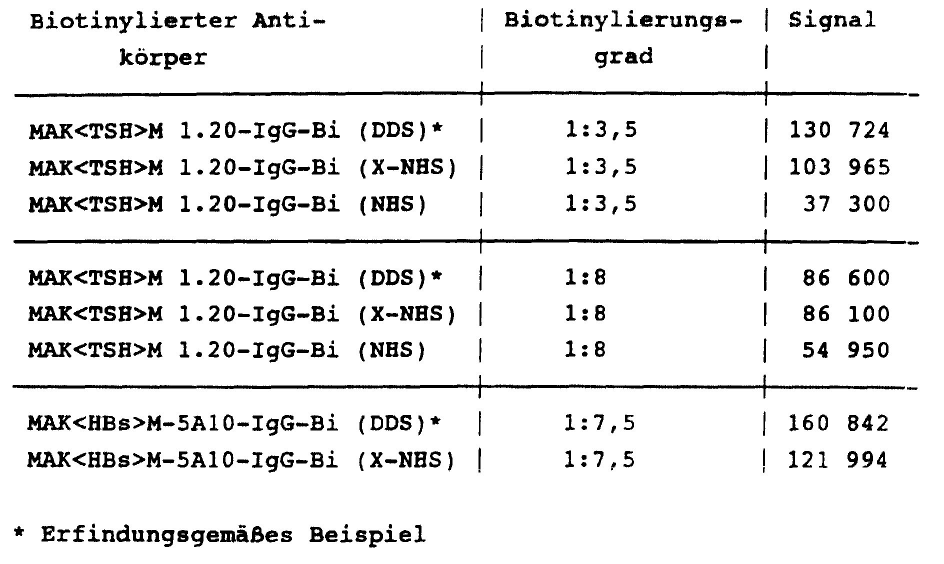

Die Daten zeigen, daß bei der Elektrochemilumineszenz-Messung über mit Biotin-DDS bzw. Biotin-X-NHS biotinylierten Antikörpern deutlich höhere Signale als bei Biotinylierung mit dem spacerfreien Biotin-NHS erhalten werden. Ein niedrigerer Biotinylierungsgrad ergibt jeweils höhere Signale. Mit den über Biotin-DDS biotinylierten Antikörpern wird dabei eine nochmals um 25,7 % höhere Signalausbeute erhalten als mit den über Biotin-X-NHS biotinylierten Antikörpern. Ein Signaloptimum bei niedrigem Biotinylierungsgrad ist günstiger, da der biotinylierte Antikörper dann in seinen Oberflächeneigenschaften weniger verändert ist als bei höherem Biotinylierungsgrad.

### Beispiel 6

Immunologische Bestimmung von TSH mit Streptavidin-Magnet-Partikel und photometrischer Signalmessung.

Der Nachweis von TSH erfolgt für photometrische Messung über Anti-TSH-M-A8-Fab-Fragmente, die an Peroxidase (POD) gekoppelt sind. Dieses Konjugat sowie TSH-Standard, Waschlösung und ABTS-Substrat werden einer Packung Enzymun TSH (Boehringer Mannheim GmbH, Katalog-Nr. 1488635) entnommen.

### Durchführung des Tests:

Die gemäß Beispiel 4 biotinylierten Antikörper Anti-TSH-M-1.20 werden in einer Konzentration von 3 µg/ml in Inkubationspuffer gelöst.

### Inkubationspuffer: (Gewichtsangaben pro 1 l)

| | |
|---|---|
| NaH₂PO₄ | 2,5 g |
| Na₂HPO₄ | 12,8 g |
| Di-Na-Tartrat | 46 g |
| Synperonic F 68 | 5 g |
| Rinder-IgG | 1 g |
| Rinder-Albumin | 1 g |
| Phenol | 0,1 g |

Der pH-Wert wird mit NaOH auf 7,4 eingestellt.

Das Konzentrat des Enzymkonjugats MAK〈TSH〉M-A8-Fab-POD aus der Enzymun TSH-Packung wird 1:50 in Inkubationspuffer verdünnt.

Streptavidin-Magnetpartikel (Dynabeads M 280) werden in einer Konzentration von 600 µg/ml in Inkubationspuffer suspendiert und durch Schütteln in homogener Verteilung gehalten.

Vertiefungen einer Mikrotiterplatte (Nunc Maxi Sorp) werden zur Absättigung von adsorptiven Bindungsstellen mit 1 %iger Lösung von Crotein C in 50 mM HEPES-Puffer/0,15 M NaCl, pH 7,9 1 Stunde bei Raumtemperatur inkubiert.
Nach Aussaugen der Lösung werden in die Vertiefungen pipettiert:
50 µl Magnetpartikel-Suspension
50 µl Konjugatlösung MAK-A8-POD
50 µl biotinylierter Antikörper
50 µl TSH Standardlösung (40 µU)

Die Reaktionsgemische werden 45 min bei Raumtemperatur unter Schütteln inkubiert.
Anschließend werden die Magnetpartikel jeder Vertiefung mit einem passenden Magnet am Boden gesammelt, der jeweilige überstand abgegossen und je 0,3 ml Waschlösung bei entferntem Magneten auf die Partikel pipettiert.
Nach erneuter Sammlung der Partikel mit dem Magneten werden die Waschlösungen abgegossen. Der Waschvorgang wird 2 x wiederholt.
Am Ende der dritten Waschung wird in jede Vertiefung 0,2 ml ABTS-Substratlösung zugegeben und bei Raumtemperatur unter Schütteln inkubiert.
Nach 16 min wird in einem Photometer für Mikrotiterplatten die Farbextinktion in den Vertiefungen bei 405 nm gemessen.

### Das Ergebnis dieser Messungen zeigt die folgende Tabelle

| Biotinylierter Antikörper | Biotinylierungsgrad | Extinktion bei 405 nm |
|---|---|---|
| MAK〈TSH〉M-1.20-IgG-Bi(DDS) | 1:3,5 | 1,484 |
| MAK〈TSH〉M-1.20-IgG-Bi(X-NHS) | 1:3,5 | 1,225 |
| MAK〈TSH〉M-1.20-IgG-Bi(DDS) | 1:8 | 1,400 |
| MAK〈TSH〉M-1.20-IgG-Bi(X-NHS) | 1:8 | 1,291 |

Die Daten zeigen, daß in einem Magnetpartikel-Immuntest mit Antikörper-POD-Konjugat auch bei photometrischer Messung mit Biotin-DDS markierter Antikörper signifikant höhere Signale ergibt als Antikörper, der mit Biotin-X-NHS biotinyliert ist. Biotin-DDS ergibt auch wieder ein Signaloptimum bei niedrigerem Biotinylierungsgrad.

## Patentansprüche

1. Verbindung der allgemeinen Formel (I): 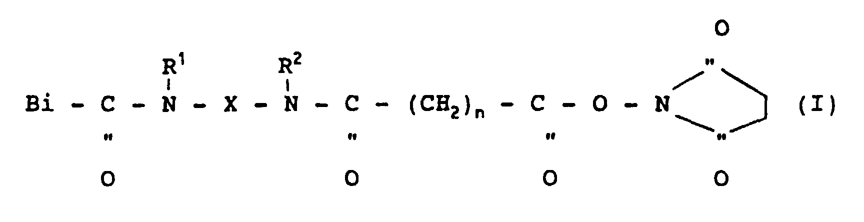 worin
Bi einen durch Abspaltung einer Carboxylgruppe von Biotin oder einem Biotinderivat stammenden Rest bedeutet, R¹ und R² unabhängig voneinander Wasserstoff oder C₁-C₄-Alkyl bedeuten,
n eine ganze Zahl von 4 bis 10 bedeutet, und
X einen durch ein oder mehrere O- oder/und S-Atome substituierten Alkylenrest mit einer Kettenlänge von 5 bis 20 Atomen bedeutet.

2. Verbindung nach Anspruch 1,
**dadurch gekennzeichnet,**
daß Bi einen von Biotin, Dethiobiotin oder Iminobiotin stammenden Rest bedeutet.

3. Verbindung nach Anspruch 1 oder 2,
**dadurch gekennzeichnet,**
daß R¹ und R² Wasserstoff bedeuten.

4. Verbindung nach einem der Ansprüche 1 bis 3,
**dadurch gekennzeichnet,**
daß n eine ganze Zahl von 6 bis 8 bedeutet.

5. Verbindung nach einem der Ansprüche 1 bis 4,
**dadurch gekennzeichnet,**
daß X einen durch ein oder mehrere O-Atome substituierten Alkylenrest darstellt.

6. Verbindung nach einem der Ansprüche 1 bis 5,
**dadurch gekennzeichnet,**
daß X einen Rest (CH₂-CH₂O)ₘ CH₂-CH₂ darstellt, wobei m eine ganze Zahl von 1 bis 5 bedeutet.

7. Verbindung nach einem der Ansprüche 1 bis 6,
**dadurch gekennzeichnet,**
daß X einen Rest mit einer Kettenlänge von 5 bis 11 Atomen bedeutet.

8. Biotinoyl-amino-3,6-dioxaoctanyl-aminocarbonylheptansäure-N-hydroxysuccinimidester.

9. Verfahren zur Herstellung einer Verbindung nach einem der Ansprüche 1 bis 8,
**dadurch gekennzeichnet,**
daß man eine Verbindung der allgemeinen Formel (II): 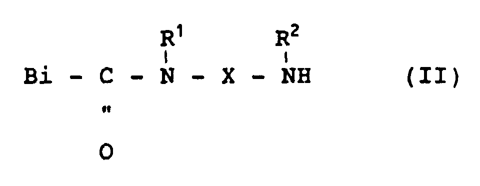
in einem wasserfreien aprotischen Lösungsmittel und in Gegenwart einer Base mit einer Verbindung der allgemeinen Formel (III): 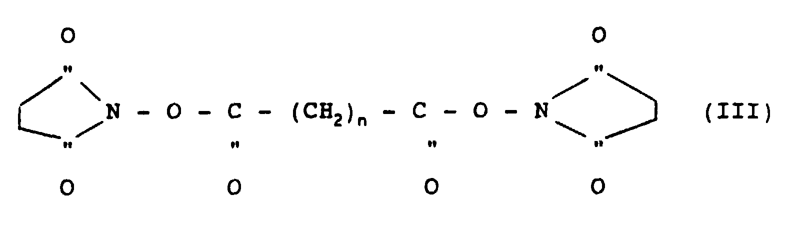
umsetzt und das Reaktionsprodukt gewinnt, wobei Bi, R¹, R², X und n die in Anspruch 1 angegebene Bedeutung besitzen.

10. Verwendung einer Verbindung nach einem der Ansprüche 1 bis 8 zur Biotinylierung von Substanzen mit freien primären oder/und sekundären Aminogruppen.

11. Verwendung nach Anspruch 10 zur Biotinylierung von Proteinen, Peptiden, Nukleinsäuren, Nukleotiden oder Haptenen.

12. Konjugat der allgemeinen Formel (IV): 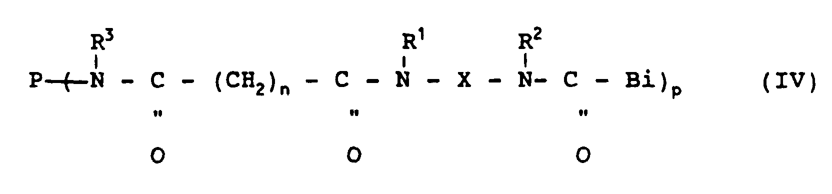 wobei
P eine Substanz bedeutet, die mindestens eine primäre oder/und sekundäre Aminogruppe NHR³ besitzt,
R³ Wasserstoff oder/und einen gegebenenfalls substituierten Alkylrest bedeutet,
p eine ganze Zahl von ≥ 1 bedeutet und
Bi, R¹, R², X und n wie in Anspruch 1 definiert sind.

13. Konjugat nach Anspruch 11,
**dadurch gekennzeichnet,**
daß P ein Protein, ein Peptid, eine Nukleinsäure, ein Nukleotid oder ein Hapten ist.

14. Konjugat nach Anspruch 13,
**dadurch gekennzeichnet,**
daß P ein Protein ist.

15. Konjugat nach Anspruch 14,
**dadurch gekennzeichnet,**
daß P ein Antikörper oder ein enzymatisch aktives Protein ist.

16. Verfahren zur Bestimmung eines Analyten unter Verwendung eines Konjugats nach einem der Ansprüche 12 bis 15.

17. Reagenz zur Bestimmung eines Analyten,
**dadurch gekennzeichnet,**
daß es ein Konjugat nach einem der Ansprüche 12 bis 15 enthält.

18. Reagenz nach Anspruch 17,
**dadurch gekennzeichnet,**
daß es in Form einer Lösung, eines Pulvers oder eines Lyophilisats ist.

## Claims

1. Compound of the general formula (I): 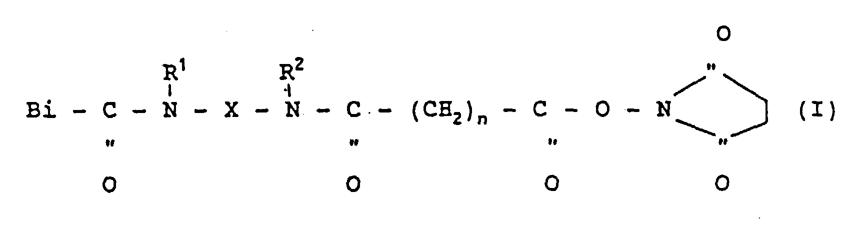 in which
Bi denotes a residue derived by cleavage of a carboxyl group from biotin or from a biotin derivative,
R¹ and R² denote independently of one another hydrogen or C₁-C₄ alkyl,
n denotes an integer from 4 to 10 and
X denotes an alkylene residue with a chain length of 5 to 20 atoms substituted by one or several O or/and S atoms.

2. Compound as claimed in claim 1,
**wherein**
Bi denotes a residue derived from biotin, dethiobiotin or iminobiotin.

3. Compound as claimed in claim 1 or 2,
**wherein**
R¹ and R² denote hydrogen.

4. Compound as claimed in one of the claims 1 to 3,
**wherein**
n denotes an integer from 6 to 8.

5. Compound as claimed in one of the claims 1 to 4,
**wherein**
X represents an alkylene residue substituted by one or several O atoms.

6. Compound as claimed in one of the claims 1 to 5,
**wherein**
X represents a residue (CH₂-CH₂O)ₘCH₂-CH₂ in which m denotes an integer from 1 to 5.

7. Compound as claimed in one of the claims 1 to 6,
**wherein**
X denotes a residue with a chain length of 5 to 11 atoms.

8. Biotinoyl-amino-3,6-dioxaoctanyl-aminocarbonylheptanoic acid-N-hydroxysuccinimide ester.

9. Process for the production of a compound as claimed in one of the claims 1 to 8,
**wherein**
a compound of the general formula (II): 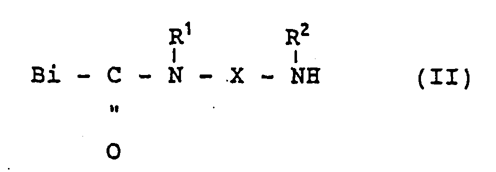 is reacted in an anhydrous aprotic solvent and in the presence of a base with a compound of the general formula (III): 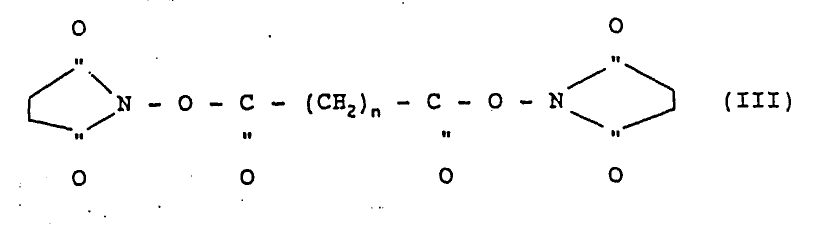 and the reaction product is isolated, in which Bi, R¹, R², X and n have the meanings stated in claim
1.

10. Use of a compound as claimed in one of the claims 1 to 8 for biotinylating substances with free primary or/and secondary amino groups.

11. Use as claimed in claim 10 for biotinylating proteins, peptides, nucleic acids, nucleotides or haptens.

12. Conjugate of the general formula (IV): 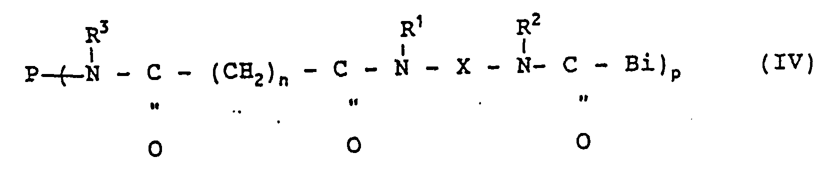 in which
P denotes a substance which has at least one primary or/and secondary amino group NHR³, R³ denotes hydrogen or/and an alkyl residue which is substituted if desired,
p denotes an integer of ≥ 1 and
Bi, R¹, R², X and n are defined as in claim 1.

13. Conjugate as claimed in claim 11,
**wherein**
P is a protein, a peptide, a nucleic acid, a nucleotide or a hapten.

15. Conjugate as claimed in claim 13,
**wherein**
P is a protein.

16. Conjugate as claimed in claim 14,
**wherein**
P is an antibody or an enzymatically active protein.

17. Method for the determination of an analyte using a conjugate as claimed in one of the claims 12 to 15.

18. Reagent for the determination of an analyte,
**wherein**
it contains a conjugate as claimed in one of the claims 12 to 15.

19. Reagent as claimed in claim 17,
**wherein**
it is in the form of a solution, a powder or a lyophilisate.

## Revendications

1. Composé de formule générale (I): 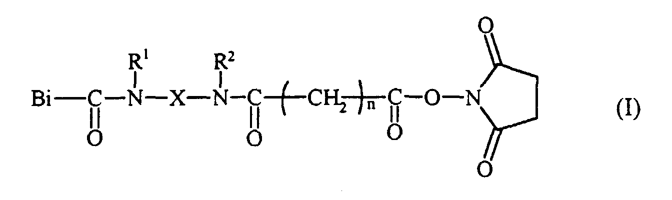 dans laquelle
Bi représente un reste dérivé de la biotine ou d'un dérivé de biotine par élimination d'un groupe carboxyle,
R¹ et R² représentent, indépendamment l'un de l'autre, un atome d'hydrogène ou un reste alkyle en C₁-C₄,
n est un nombre entier de 4 à 10, et
X représente un reste alkylène substitué par un ou plusieurs atomes de O et/ou S, ayant une longueur de chaîne de 5 à 20 atomes.

2. Composé selon la revendication 1, caractérisé en ce que Bi représente un reste dérivé de biotine, de déthiobiotine ou d'iminobiotine.

3. Composé selon la revendication 1 ou 2, caractérisé en ce que R¹ et R² représentent l'hydrogène.

4. Composé selon l'une des revendications 1 à 3, caractérisé en ce que n est un nombre entier de 6 à 8.

5. Composé selon l'une des revendications 1 à 4, caractérisé en ce que X représente un reste alkylène substitué par un ou plusieurs atomes de O.

6. Composé selon l'une des revendications 1 à 5, caractérisé en ce que X représente un reste (CH₂-CH₂O)ₘCH₂-CH₂, dans lequel m est un nombre entier de 1 à 5.

7. Composé selon l'une des revendications 1 à 6, caractérisé en ce que X est un reste ayant une longueur de chaîne de 5 à 11 atomes.

8. Biotinoylamino-3,6-dioxaoctanylaminocarbonylheptanoate de N-hydroxysuccinimide.

9. Procédé de préparation d'un composé selon l'une des revendications 1 à 8, caractérisé en ce que l'on fait réagir un composé de formule générale (II): 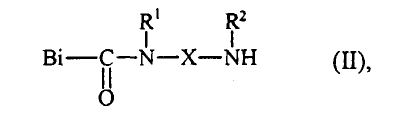 dans un solvant aprotique anhydre et en présence d'une base, avec un composé de formule générale (III) 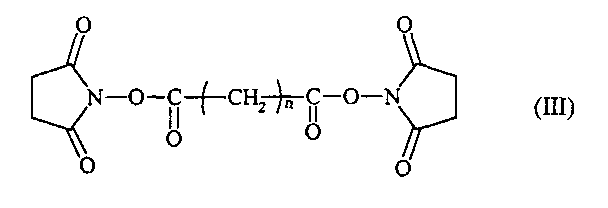 et on récupère le produit de la réaction, Bi, R¹, R², X et n ayant la signification indiquée dans la revendication 1.

10. Utilisation d'un composé selon l'une des revendications 1 à 8 pour la biotinylation de substances ayant des groupes amino primaires et/ou secondaires libres.

11. Utilisation selon la revendication 10 pour la biotinylation de protéines, de peptides, d'acides nucléiques, de nucléotides ou d'haptènes.

12. Conjugué de formule générale (IV): 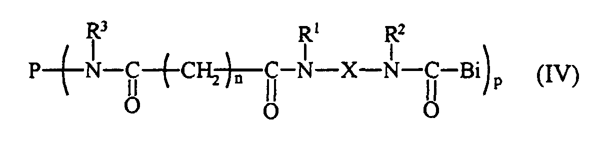 dans laquelle
P est une substance contenant au moins un groupe amino primaire et/ou secondaire NHR³,
R³ représente un atome d'hydrogène et/ou un reste alkyle éventuellement substitué,
p est un nombre entier supérieur ou égal à 1 et
Bi, R¹, R², X et n ont la définition indiquée dans la revendication 1.

13. Conjugué selon la revendication 11, caractérisé en ce que P est une protéine, un peptide, un acide nucléique, un nucléotide ou un haptène.

14. Conjugué selon la revendication 13, caractérisé en ce que P est une protéine.

15. Conjugué selon la revendication 14, caractérisé en ce que P est un anticorps ou une protéine à activité enzymatique.

16. Procédé de détermination d'un analyte utilisant un conjugué selon l'une des revendications 12 à 15.

17. Réactif pour la détermination d'un anatyte, caractérisé en ce qu'il contient un conjugué selon l'une des revendications 12 à 15.

18. Réactif selon la revendication 17, caractérisé en ce qu'il est sous forme d'une solution, d'une poudre ou d'un produit lyophilisé.
